(19) Europäisches Patentamt / European Patent Office / Office européen des brevets

(11) **EP 4 177 583 B1**

(12) **EUROPEAN PATENT SPECIFICATION**

(45) Date of publication and mention
of the grant of the patent:
**11.02.2026 Bulletin 2026/07**

(21) Application number: **20944316.7**

(22) Date of filing: **24.12.2020**

(51) International Patent Classification (IPC):
**G01K 1/02** *(2021.01)*      **G01K 7/00** *(2006.01)*
**G01K 15/00** *(2006.01)*     **A61B 5/01** *(2006.01)*
**G01K 3/10** *(2006.01)*      **G01K 7/42** *(2006.01)*
**A61B 5/00** *(2006.01)*      **G01J 5/00** *(2022.01)*
**G01K 13/20** *(2021.01)*     **G01J 5/02** *(2022.01)*

(52) Cooperative Patent Classification (CPC):
**G01K 3/10; A61B 5/01; A61B 5/7275; A61B 5/746;
G01K 7/42; G01K 13/20;** A61B 5/0008;
A61B 2560/0252; G01J 5/0025; G01J 5/026

(86) International application number:
**PCT/CN2020/139177**

(87) International publication number:
**WO 2022/007348 (13.01.2022 Gazette 2022/02)**

(54) **TEMPERATURE DETECTION METHOD, COMPUTER-READABLE STORAGE MEDIUM, AND ELECTRONIC DEVICE**

VERFAHREN ZUR TEMPERATURERFASSUNG, COMPUTERLESBARES SPEICHERMEDIUM UND ELEKTRONISCHE VORRICHTUNG

PROCÉDÉ DE DÉTECTION DE TEMPÉRATURE, SUPPORT D'INFORMATIONS LISIBLE PAR ORDINATEUR ET DISPOSITIF ÉLECTRONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.07.2020 CN 202010642012**

(43) Date of publication of application:
**10.05.2023 Bulletin 2023/19**

(73) Proprietor: **Zhejiang Uniview Technologies Co., Ltd.
Hangzhou, Zhejiang 310051 (CN)**

(72) Inventor: **WANG, Hui
Hangzhou, Zhejiang 310051 (CN)**

(74) Representative: **Plougmann Vingtoft a/s
Strandvejen 70
2900 Hellerup (DK)**

(56) References cited:
CN-A- 1 584 523       CN-A- 1 584 523
CN-A- 101 435 727     CN-A- 103 181 757
CN-A- 105 371 968     CN-A- 105 371 968
CN-A- 108 201 440     US-A- 4 843 577
US-A1- 2018 008 149

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

The present application relates to the technical field of temperature detection, in particular to a temperature detection method, a computer-readable storage medium and an electronic device.

BACKGROUND

With the rapid development of science and technology, people pay more and more attention to safety of human body. In some scenarios, it is very important to perform non-contact temperature measurement on the human body.

The non-contact human body temperature measurement method uses a forehead thermometer for human body temperature measurement. In the outdoor scene, since the body surface temperature of the human body fluctuates greatly due to the influence of the environment, it takes a certain period of time to restore the normal body surface temperature, which leads to the temperature measurement results obtained by the forehead thermometer often not being able to truly reflect temperature of the human body. Due to the limitations of some scenarios, such as the entrances and exits of stations, shopping malls, and office buildings, it is not possible to test everyone for a sufficiently long time, and the accuracy of the temperature test results cannot be guaranteed.

Further relevant technologies are also known from US 4843577 A (MURAMOTO YUTAKA [JP]) 27 June 1989 (1989-06-27) which relates to ELECTRONIC CLINICAL THERMOMETER.

Further relevant technologies are also known from CN 1584523 A (OMRON HEALTHCARE CO LTD [JP]) 23 February 2005 (2005-02-23) which relates to ELECTRONIC CLINICAL THERMOMETER.

Further relevant technologies are also known from CN 105 371 968 A (GUANGZHOU CVTE ELECTRONIC TECH) 2 March 2016 (2016-03-02) which relates to ELECTRONIC THERMOMETER CONTROL METHOD AND DEVICE.

SUMMARY

The present invention is set out in the appended set of claims.

BRIEF DESCRIPTION OF DRAWINGS

FIG. 1 is a flow chart of a temperature detection method according to an embodiment of the present application;

FIG. 2 is a schematic diagram of a temperature trend detection window according to an embodiment of the present application;

FIG. 3 is a schematic diagram of a temperature trend detection window according to an embodiment of the present application;

FIG. 4 is a schematic structural diagram of a temperature detection apparatus according to an embodiment of the present application;

FIG. 5 is a schematic structural diagram of an electronic device according to an embodiment of the present application.

DETAILED DESCRIPTION

The present application is described hereinafter in conjunction with the drawings and embodiments. The embodiments described here are merely used to explain the present application rather than limiting the present application. For ease of description, only some structures rather than all structures related to the present application are shown in the drawings.

Some exemplary embodiments are described as processes or methods depicted as flowcharts. Although the flowcharts describe steps as being sequential performed, many of the steps may be performed in parallel, concurrently, or simultaneously. Additionally, the order of various steps may be rearranged. The process may be terminated when its operations are completed, but may also have additional steps not included in the drawings. The processing may correspond to a method, function, procedure, subroutine, subprogram, or the like.

There is a corresponding relationship between a body surface temperature and a body temperature of a human body, based on the corresponding relationship, the corresponding human body temperature may be calculated by measuring the body surface temperature. The forehead thermometers and ear thermometers sold on the market are all based on this principle.

The body surface temperature of the human body will change with changes in factors such as ambient temperature, air humidity, ambient wind force, body surface clothing coverage of human body, and movement status of human body. Therefore, when the above factors change, the phenomenon of inaccurate temperature measurement may occur.

For example, when a person walks from a high-temperature indoor environment to a relatively low-temperature gate for temperature measurement, the body surface temperature of the human body may be relatively high, and when a person walks from a low-temperature outdoor environment to an indoor environment for temperature measurement, the body surface temperature of the human body may be relatively low. The body surface temperatures of the human body in these cases are in a state of non-heat balance, and measuring the temperature of the body surface at these times, relatively inaccurate temperature values may be obtained.

The body surface temperature of the human body is affected by heat production in the body and heat dissipation through the body surface. The heat production methods of the human body include basal metabolic heat production, heat production by exercise, heat production by human vaso contraction and relaxation, and heat production by human body tremor, etc. The human body heat dissipation methods include heat dissipation by conduction, heat dissipation by convection, heat dissipation by radiation, and heat dissipation by evaporation. When the above-mentioned factors affecting heat production and heat dissipation appear, they will all lead to changes in the body surface temperature of the human body. When the heat produced in the body is more than the heat dissipated from the body surface, the body surface temperature rises. When the heat produced in the body is less than the heat dissipated from the body surface, the body surface temperature will decline. When the heat produced in the human body is equal to the heat dissipated from the body surface, the body surface temperature will maintain a relatively constant value. At this time, the body surface temperature may represent the body temperature relatively accurately.

In order to better measure the temperature of the human body, it is necessary to implement different temperature measurement strategies according to the changing trend of the body surface temperature to ensure the accuracy of the measurement results.

In the practical temperature measurement application, the temperature measurement speed is also an important indicator, which will affect the practical use experience. For example, when the temperature measurement is clocked in during the peak commute, the low temperature measurement speed will cause the queue to be too long, which, in one aspect, will affect the speed of getting on and off work, and in another aspect, will lead to gathering of a large number of people and increase the risk of cross-infection.

Therefore, it is necessary to integrate the body temperature changing trend and the temperature measurement speed to obtain a temperature measurement solution that can take both aspects into consideration. In this solution, the body temperature detection is to predict the body temperature by detecting the body surface temperature, and all the temperatures described in the following process are the body surface temperature.

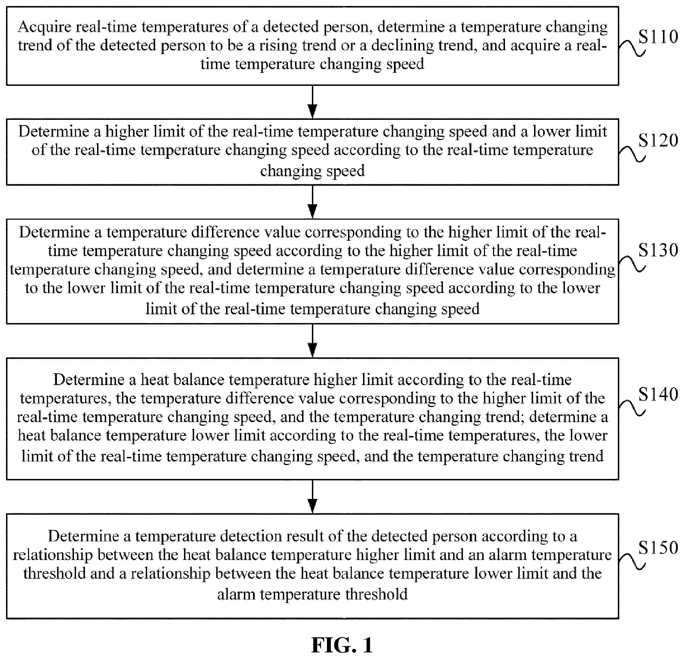

FIG. 1 is a flow chart of a temperature detection method according to an embodiment of the present application. This embodiment may be applied to the situation of body temperature detection. This method may be executed by a temperature detection apparatus according to an embodiment of the present application. The temperature detection apparatus may be implemented by means of software and/or hardware, and may be integrated into electronic devices with corresponding computation capabilities.

As shown in FIG. 1, the temperature detection method includes: S110, S120, S130, S140 and S150.

In S110, real-time temperatures of a person is acquired, a temperature changing trend of the person is determined to be a rising trend or a declining trend, and a real-time temperature changing speed is acquired.

The person may be a staff, a student and a traveler. The real-time temperature of the person may be obtained through a temperature gun, etc. The real-time temperature of the person may be one temperature value or multiple temperature values, for example, if the body temperature gun is controlled to collect body temperatures according to a preset frequency, multiple body temperature values may be obtained, for example, if temperature values are collected once every 0.2 seconds, then in a two-second acquisition time, ten real-time temperature values may be obtained.

After the temperature values of multiple real-time temperatures are collected, the temperature changing trend of the person may be determined according to the change rules of the temperature values of the multiple real-time temperatures. The temperature changing trend may include a rising trend, a declining trend, and a flat trend. If the body temperature of the person is on a flat trend, it means that the measured real-time temperature are consistent with the actual heat balance temperature of the person, and a determination can be made directly.

The real-time temperature changing speed may be determined according to one or more slopes between multiple temperature values, for example, may be determined according to an average value of slopes between two adjacent values of the multiple temperature values. For example, a slope formed by a temperature value 1 and a temperature value 2, a slope formed by the temperature value 2 and a temperature value 3, and so on, and then all obtained slopes are averaged to obtain the real-time temperature changing speed.

In this solution, since the multiple temperature values collected are analyzed to determine the temperature changing rule, there may also be a case where multiple temperature values are unstable, for example, the rising trends and the falling trends appear alternately. In this case, a reminder can be given to inform the staff to re-detect the temperature of the person or to check the connection and working conditions of the device to eliminate the errors caused by the device and the errors caused by human manipulation of the physical objects during the collection process.

In this embodiment, optionally, the step of acquiring real-time temperatures of a person and determining a temperature changing trend of the person to be a rising trend or a declining trend includes the following.

The real-time temperatures of the person are sampled according to a preset period; an average slope of real-time temperature sampled points of the person is determined through a preset temperature trend detection window; and if the average slope is greater than 0, it is determined that the temperature changing trend is the rising trend; if the average slope is less than 0, it is determined that the temperature changing trend is the declining trend.

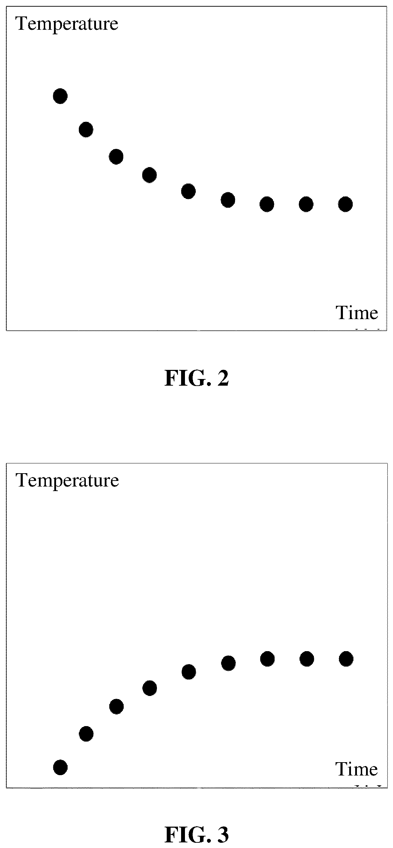

The preset period may be 0.2 seconds, 0.5 seconds, or a longer or shorter time. After sampling, the temperature sampled points may be displayed in the temperature trend detection window. Specifically, the temperature trend detection window may be provided in the temperature gun, and may also be provided in an intelligent terminal, such as an intelligent mobile terminal, a computer, and a tablet computer, connected in a communication manner to the temperature gun. FIG. 2 and FIG. 3 are schematic diagrams of the temperature trend detection window according to an embodiment of the present application. As shown in FIG. 2 and FIG. 3, if the temperature gradually increases over time, the average slope is greater than 0, it indicates that the temperature changing trend of the body surface temperature of the person is the rising trend, and if the average slope is less than 0, then it indicates that the temperature changing trend of the body surface temperature of the person is the declining trend.

On the basis of the above technical solution, optionally, the method further includes the following.

If the temperature changing trend is the rising trend, then a temperature increasing speed in the temperature trend detection window is calculated and used as the real-time temperature changing speed; and if the temperature changing trend is the declining trend, a temperature decreasing speed in the temperature trend detection window is calculated and used as the real-time temperature changing speed.

In the rising trend and the declining trend, the temperature increasing speed or temperature decreasing speed in the temperature trend detection window may be calculated as the real-time temperature changing speed.

For example, if the temperature is on a rising trend, an average temperature increasing speed SpeedTempInc in the temperature trend detection window is calculated.

If the temperature is on a declining trend, an average temperature decreasing speed SpeedTempDec in the temperature trend detection window is calculated.

In S120, a higher limit of the real-time temperature changing speed and a lower limit of the real-time temperature changing speed are determined according to the real-time temperature changing speed.

The higher limit of the real-time temperature changing speed and the lower limit of the real-time temperature changing speed may be determined according to the real-time temperature changing speed and a preset empirical coefficient.

Optionally, the real-time temperature changing speed includes a temperature increasing speed and a temperature decreasing speed.

The determining a higher limit of the real-time temperature changing speed and a lower limit of the real-time temperature changing speed according to the real-time temperature changing speed includes the following.

The higher limit of the real-time temperature changing speed is determined according to the products of the temperature increasing speed and a preset higher limit coefficient; and the lower limit of the real-time temperature changing speed is determined according to the products of the temperature decreasing speeds and a preset lower limit coefficient.

In this embodiment, optionally, the determining a higher limit of the real-time temperature changing speed and a lower limit of the real-time temperature changing speed according to the real-time temperature changing speed includes the following.

The higher limit of the real-time temperature changing speed is determined according to the products of the real-time temperature changing speed and a preset higher limit coefficient; and the lower limit of the real-time temperature changing speed is determined according to the products of the real-time temperature changing speed and a preset lower limit coefficient.

The absolute value SpeedTemp of the temperature changing speed SpeedTempInc or SpeedTempDec may be taken to calculate the higher limit of the real-time temperature changing speed SpeedTempLow and the lower limit of the real-time temperature changing speed SpeedTempHig:

$$SpeedTempLow = SpeedTemp*KLow;$$

$$SpeedTempHig = SpeedTemp*KHig;$$

The KLow is an empirical coefficient less than 1, and KHig is an empirical coefficient greater than 1.

Controlling the temperature changing speed through the empirical coefficient can achieve the effect that the heat balance temperature is calculated through a range value, which can ensure that the finally analyzed heat balance temperature is more accurate.

In S130, a temperature difference value corresponding to the higher limit of the real-time temperature changing speed is determined according to the higher limit of the real-time temperature changing speed, and a temperature difference value corresponding to the lower limit of the real-time temperature changing speed is determined according to the lower limit of the real-time temperature changing speed.

The temperature difference value is the difference between a heat balance temperature and a real-time temperature. The temperature difference value corresponding to the higher limit of the real-time temperature changing speed and the temperature difference value corresponding to lower limit of the real-time temperature changing speed are determined according to the higher limit of the real-time temperature changing speed, the lower limit of the real-time temperature changing speed and the changing speed low limit.

In this technical solution, the characteristic that when the temperature difference value is larger, the changing speed is higher can be utilized to construct a table of the mapping relationship between the magnitudes of the changing speed and the temperature difference values. The respective temperature difference values corresponding to an obtained higher limit of the real-time temperature changing speed and an obtained lower limit of the real-time temperature changing speed are determined by looking up from the table according to the obtained higher limit of the real-time temperature changing speed and the obtained lower limit of the real-time temperature changing speed.

The lower limit of the real-time temperature changing speed SpeedTempLow and the higher limit of the real-time temperature changing speed SpeedTempHig are used to obtain temperature difference values TempDiffCurLow and TempDiffCurHigh at the current speed respectively by looking up a table using the linear interpolation method.

In S140, a heat balance temperature higher limit is determined according to the real-time temperatures, the temperature difference value corresponding to the higher limit of the real-time temperature changing speed, and the temperature changing trend; a heat balance temperature lower limit is determined according to the real-time temperatures, the lower limit of the real-time temperature changing speed, and the temperature changing trend.

In this solution, the heat balance temperature may be determined according to the temperature changing trend, the currently measured real-time temperature, the temperature difference value corresponding to the higher limit of the real-time temperature changing speed determined above and the temperature difference value corresponding to the lower limit of the real-time temperature changing speed determined above.

According to the invention, the determining a heat balance temperature higher limit and a heat balance temperature lower limit according to the real-time temperatures and the temperature difference value corresponding to the higher limit of the real-time temperature changing speed and the temperature difference value corresponding to the lower limit of the real-time temperature changing speed, and according to the temperature changing trend includes the following.

When the temperature changing trend is the rising trend, the heat balance temperature higher limit is the sum of the real-time temperature and the temperature difference value corresponding to the higher limit of the real-time temperature changing speed; and the heat balance temperature lower limit is the sum of the real-time temperature and the temperature difference value corresponding to the lower limit of the real-time temperature changing speed.

When the temperature changing trend is the declining trend, the heat balance temperature higher limit is a difference between the real-time temperature and the temperature difference value corresponding to the lower limit of the real-time temperature changing speed; and the heat balance temperature lower limit is a difference between the real-time temperature and the temperature difference value corresponding to the higher limit of the real-time temperature changing speed.

According to TempDiffCurLow, TempDiffCurHigh, and the current temperature TempCur, the temperature values under Heat balance TempHeatBalanceLow and TempHeatBalanceHig are calculated.

If the temperature is on a rising trend, then

$$TempHeatBalanceLow = TempCur + TempDiffCurLow;$$

and

$$TempHeatBalanceHig = TempCur + TempDiffCurHig.$$

If the temperature is on a declining trend, then

$$TempHeatBalanceLow = TempCur - TempDiffCurHig;$$

and

$$TempHeatBalanceHig = TempCur - TempDiffCurLow.$$

Through the above calculations, the heat balance temperature higher limit and the heat balance temperature lower limit may be obtained respectively for different temperature changing trends.

In S150, a temperature detection result of the person is determined according to a relationship between the heat balance temperature higher limit and an alarm temperature threshold and a relationship between the heat balance temperature lower limit and the alarm temperature threshold.

The body temperature threshold for human body fever alarm is generally 37.3 degrees, but the corresponding body surface temperatures under different ambient temperatures are different, therefore, it is necessary to establish a table of mapping relationship of ambient temperatures to alarm body surface temperatures.

The table of mapping relationship of the ambient temperatures to the alarm body surface temperatures may be established through experiments.

According to the current ambient temperature TempEnv, the alarm temperature threshold ThrTempAlm under the current ambient temperature is acquired by looking up the table through the linear interpolation method. For example, if the ambient temperature is 20 degrees, the alarm body surface temperature threshold may be lower than 37.3 degrees, such as 37 degrees.

Here, it may be divided into two cases, namely a rising trend and a declining trend.

The object of this embodiment is to detect the person whose heat balance temperature exceeds the alarm temperature threshold, that is, to detect the person with high fever. Therefore, on the rising trend, the heat balance temperature lower limit corresponding to the lower limit of the real-time temperature changing speed may be determined. If the heat balance temperature lower limit exceeds the alarm temperature threshold, it is determined that the person is in a state of high fever. In the declining stage, the heat balance temperature lower limit corresponding to the higher limit of the real-time temperature changing speed may be determined, and if the heat balance temperature lower limit exceeds the alarm temperature threshold, it is determined that the person is in a state of high fever

In this embodiment, the determining a temperature detection result of the person according to a relationship between the heat balance temperature higher limit and an alarm temperature threshold and a relationship between the heat balance temperature lower limit and the alarm temperature threshold, includes the following.

When the temperature changing trend is the rising trend, if the alarm temperature threshold is less than the heat balance temperature lower limit, it is determined that the temperature detection result is an abnormal high temperature; and if the alarm temperature threshold is greater than the heat balance temperature higher limit, it is determined that the temperature detection result is a normal body temperature.

When the temperature changing trend is the declining trend, if the alarm temperature threshold is less than the heat balance temperature lower limit, it is determined that the temperature detection result is an abnormal high temperature; and if the alarm temperature threshold is greater than the heat balance temperature higher limit, it is determined that the temperature detection result is a normal body temperature.

With such a setting, it is determined whether the person is in a state of high fever according to the magnitude relationship between the alarm temperature threshold and the heat balance temperature limits.

In the technical solution according to an embodiment of the present application, the real-time temperatures of the person are acquired; and it is determined that the temperature changing trend of the person is a rising trend or a declining trend, and the real-time temperature changing speed is acquired; the higher limit of the real-time temperature changing speed and the lower limit of the real-time temperature changing speed are determined according to the real-time temperature changing speed; the temperature difference value corresponding to the higher limit of the real-time temperature changing speed and the temperature difference value corresponding to the lower limit of the real-time temperature changing speed are determined respectively according to the higher limit of the real-time temperature changing speed and the lower limit of the real-time temperature changing speed; the heat balance temperature higher limit and the heat balance temperature lower limit are determined according to the real-time temperatures and the temperature difference value corresponding to the higher limit of the real-time temperature changing speed and the temperature difference value corresponding to the lower limit of the real-time temperature changing speed; and the temperature detection result of the person is determined according to the relationship between the heat balance temperature higher limit and the alarm temperature threshold and the relationship between the heat balance temperature lower limit and the alarm temperature threshold. By adopting the technical solution according to this application, the heat balance temperature value may be calculated by performing temperature trend detection, and the human body temperature detection result can be determined according to the relationships between the alarm temperature threshold and the heat balance temperature values, so as to achieve the object of improving the accuracy of human body temperature detection.

On the basis of the above technical solution, before the determining the temperature detection result of the person according to the relationship between the heat balance temperature higher limit and an alarm temperature threshold and the relationship between the heat balance temperature lower limit and the alarm temperature threshold, the method further includes the following.

A balancing time higher limit is determined according to the higher limit of the real-time temperature changing speed and a balancing time lower limit is determined according to the lower limit of the real-time temperature changing speed.

Specifically, the balancing time higher limit and the balancing time lower limit are the parameters in the table of mapping relationship between the magnitudes of changing speed and the temperature difference values, that is, the time value required for the temperature to reach balance, and according to the time value required for the temperature to reach balance and through determination, the temperature measurement speed is ensured.

Specifically, the table of mapping relationship between the magnitudes of changing speed and the temperature difference values, the table items may be as follows.

Table item 1: temperature difference 1, temperature changing speed 1, time for temperature reaching balance 1;
Table item 2: temperature difference 2, temperature changing speed 2, time for temperature reaching balance 2;
Table item N: temperature difference N, temperature changing speed N, time for temperature reaching balance N.

The lower limit of the real-time temperature changing speed SpeedTempLow and the higher limit of the real-time temperature changing speed SpeedTempHig are used respectively to obtain temperature difference values at the current speed TempDiffCurLow and TempDiffCurHigh and balancing time values TimeHeatBalanceLow and TimeHeatBalanceHig by looking up a table using the linear interpolation method.

The temperature values under heat balance TempHeatBalanceLow and TempHeatBalanceHig are calculated according to TempDiffCurLow, TempDiffCurHigh, and current temperature TempCur.

If the temperature is on a rising trend, then TempHeatBalanceLow (temperature value under heat balance) = TempCur+TempDiffCurLow (temperature difference value under current speed); and TempHeatBalanceHig (temperature value under heat balance) = TempCur+TempDiffCurHig (temperature difference value under current speed).

If the temperature is in a declining trend, then TempHeatBalanceLow = TempCur - TempDiffCurHig; and TempHeatBalanceHig = TempCur - TempDiffCurLow.

Correspondingly, the determining a temperature detection result of the person according to a relationship between the heat balance temperature higher limit and an alarm temperature threshold and a relationship between the heat balance temperature lower limit and the alarm temperature threshold, includes the following.

When the temperature changing trend is the rising trend, if the alarm temperature threshold is greater than the heat balance temperature lower limit and less than the heat balance temperature higher limit, then a relationship between a balancing time for the latest real-time temperature to rise to the alarm temperature threshold and an acceptable measurement time threshold is determined; and if a balancing time for the latest real-time temperature to rise to the alarm temperature threshold is less than or equal to the acceptable measurement time threshold, then temperature measurement is continued; and if a balancing time for the latest real-time temperature to rise to the alarm temperature threshold is greater than the acceptable measurement time threshold, issuing a warning message of temperature instability.

When the temperature changing trend is the declining trend, if the alarm temperature threshold is greater than the heat balance temperature lower limit and less than the heat balance temperature higher limit, then determining a relationship between a balancing time for the latest real-time temperature to decline to the alarm temperature threshold and the acceptable measurement time threshold; if the time for the latest real-time temperature to decline to the alarm temperature threshold is less than or equal to the acceptable measurement time threshold, then the temperature measurement is continued; and if the time for the latest real-time temperature to decline to the alarm temperature threshold is greater than the acceptable measurement time threshold, a warning message of temperature instability is issued.

The acceptable measurement time threshold is determined according to the detection environment, for example, in a train station, due to the large number of passengers, it can often be set short, for example, 10 seconds; and if in an office, due to less cases of coming in and out of workers, it may be set long, for example, 25 seconds.

Further since the balancing times corresponding to the rising state and the declining state respectively have been determined previously, it may be determined whether to wait for the length of the balancing time before performing body temperature detection on the person according to the comparison of the balancing time with the measurement time threshold.

If it is on the rising trend, and the alarm temperature threshold is greater than the heat balance temperature lower limit and less than the heat balance temperature higher limit, then the relationship between the balancing time for rising from the latest real-time temperature to the alarm temperature threshold and the acceptable measurement time threshold is determined.

If the balancing time for rising from the latest real-time temperature to the alarm temperature threshold is less than or equal to the acceptable measurement time threshold, then the temperature measurement is continued;

If the balancing time for rising from the latest real-time temperature to the alarm temperature threshold is greater than the acceptable measurement time threshold, a warning message of temperature instability is issued.

Through such a setting, different treatments can be carried out for the person in different situations, so as to improve the accuracy of body temperature detection and the feasibility of the solution, fully consider the time efficiency of body temperature detection, and better deploy for temperature detections in various environments.

When the body temperature is on a rising trend, if the temperature is greater than the temperature alarm threshold ThrTempAlm, a high temperature alarm is warned; if the temperature is lower than the temperature alarm threshold ThrTempAlm, and it is determined if the alarm temperature threshold ThrTempAlm is less than the TempHeatBalanceLow, then a high temperature alarm is directly outputted; if the alarm temperature threshold ThrTempAlm is greater than the TempHeatBalanceHig, then a normal temperature measurement result is outputted directly; if the alarm temperature threshold ThrTempAlm is between the TempHeatBalanceLow and the TempHeatBalanceHig, and according to the time required for the temperature to reach balance TimeHeatBalanceHig when the temperature is the TempHeatBalanceHig, and according to a uniform deceleration formula, the maximum time TimeAlarmHeatBalanceMax required for the temperature to reach the alarm temperature threshold ThrTempAlm is calculated, and it is determined if the TimeAlarmHeatBalanceMax is less than or equal to the acceptable measurement time threshold ThrTimeWait, then the continuous temperature measurement state is maintained; and if the TimeAlarmHeatBalanceMax is greater than the acceptable measurement time threshold ThrTimeWait, then temperature instability is warned, and measurement is performed later.

When the body temperature is on a declining trend, if the temperature is lower than the temperature alarm threshold ThrTempAlm, a normal temperature measurement result is outputted directly; if the temperature is greater than the temperature alarm threshold ThrTempAlm, and it is determined if the alarm temperature threshold ThrTempAlm is less than the TempHeatBalanceLow, then a high temperature alarm is directly outputted; if the alarm temperature threshold ThrTempAlm is greater than the TempHeatBalanceHig, then the normal temperature measurement result is outputted directly; if the alarm temperature threshold ThrTempAlm is between the TempHeatBalanceLow and the TempHeatBalanceHig, and according to the time required for the temperature to reach balance TimeHeatBalanceLow when the temperature is the TempHeatBalanceLow, and according to a uniform deceleration formula, the time TimeAlarmHeatBalanceMax required for the temperature to reach the alarm temperature threshold ThrTempAlm is calculated, and it is determined if the TimeAlarmHeatBalanceMax is less than or equal to the acceptable measurement time threshold ThrTimeWait, then the continuous temperature measurement state is maintained; and if the TimeAlarmHeatBalanceMax is greater than the acceptable measurement time threshold ThrTimeWait, then temperature instability is warned, and measurement is performed later.

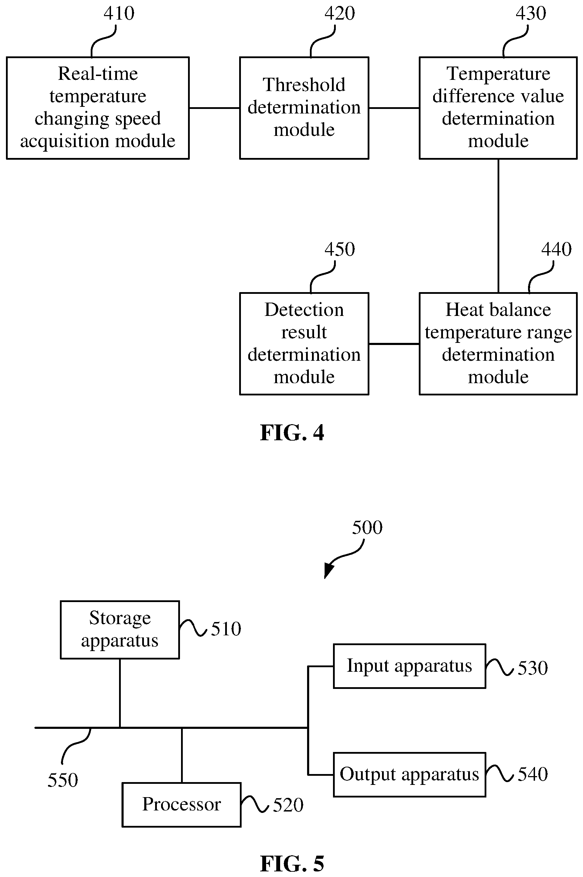

FIG. 4 is a schematic structural diagram of a temperature detection apparatus according to an embodiment of the present application. As shown in FIG. 4, the temperature detection apparatus includes: a real-time temperature changing speed acquisition module 410, a limit value determination module 420, a temperature difference value determination module 430, a heat balance temperature range determination module 440, and a detection result determination module 450.

The real-time temperature changing speed acquisition module 410 is configured to acquire real-time temperatures of a person, determine a temperature changing trend of the person to be a rising trend or a declining trend according to the real-time temperatures, and acquire a real-time temperature changing speed according to the real-time temperatures.

The limit value determination module 420 is configured to determine a higher limit of the real-time temperature changing speed and a lower limit of the real-time temperature changing speed according to the real-time temperature changing speed.

The temperature difference value determination module 430 is configured to determine a temperature difference value corresponding to the higher limit of the real-time temperature changing speed according to the higher limit of the real-time temperature changing speed and determine a temperature difference value corresponding to the lower limit of the real-time temperature changing speed according to the lower limit of the real-time temperature changing speed.

The heat balance temperature range determination module 440 is configured to determine a heat balance temperature higher limit according to the real-time temperatures, the temperature difference value corresponding to the higher limit of the real-time temperature changing speed, and the temperature changing trend; and determine a heat balance temperature lower limit according to the real-time temperatures, the lower limit of the real-time temperature changing speed, and the temperature changing trend.

The detection result determination module 450 is configured to determine the temperature detection result of the person according to a relationship between the heat balance temperature higher limit and an alarm temperature threshold and a relationship between the heat balance temperature lower limit and the alarm temperature threshold.

The above-described product can execute the methods according to the embodiments of the present application, and have corresponding functional modules and effects for executing the methods.

A storage medium containing computer-executable instructions is further provided according to the embodiments of the present application, the computer-executable instructions are used to execute a temperature detection method when executed by a computer processor, the method includes the following.

Real-time temperatures of a person are acquired, a temperature changing trend of the person is determined to be a rising trend or a declining trend according to the real-time temperatures, and a real-time temperature changing speed is acquired according to the real-time temperatures.

A higher limit of the real-time temperature changing speed and a lower limit of the real-time temperature changing speed is determined according to the real-time temperature changing speed.

A temperature difference value corresponding to the higher limit of the real-time temperature changing speed is determined according to the higher limit of the real-time temperature changing speed and a temperature difference value corresponding to the lower limit of the real-time temperature changing speed is determined according to the lower limit of the real-time temperature changing speed.

A heat balance temperature higher limit is determined according to the real-time temperatures, the temperature difference value corresponding to the higher limit of the real-time temperature changing speed, and the temperature changing trend; and a heat balance temperature lower limit is determined according to the real-time temperatures, the lower limit of the real-time temperature changing speed, and the temperature changing trend.

The temperature detection result of the person is determined according to a relationship between the heat balance temperature higher limit and an alarm temperature threshold and a relationship between the heat balance temperature lower limit and the alarm temperature threshold.

Storage media are various types of memory devices or storage devices. The "storage medium" includes: an installation medium, such as a portable compact disc read only memory (CD-ROM), a floppy disk, or a tape apparatus; a computer system memory or random access memory, such as a dynamic random access memory (DRAM), a double data rate random access memory (DDR RAM), a static random access memory (SRAM), an extended data output random access memory (EDO RAM), a Rambus (RAM), etc.; a non-volatile memory, such as a flash memory, magnetic media (for example, a hard disk or optical storage); a register or other similar types of memory elements, etc. The storage medium may further include other types of memory or combinations thereof. In addition, the storage medium may be located in a computer system in which a program is executed, or may be located in a different second computer system connected to the computer system through a network such as the Internet. The second computer system may provide program instructions to the computer for execution. The term "storage medium" may include two or more storage media that may reside in different locations, such as in different computer systems connected by a network. A storage medium may store program instructions (e.g., implemented as a computer program) executable by one or more processors.

In a storage medium containing computer-executable instructions according to an embodiment of the present application, the computer-executable instructions are not limited to perform the above-described temperature detection operations, but can also perform related operations in the temperature detection method according to any embodiment of the present application.

An electronic device is provided according to an embodiment of the present application, and the temperature detection apparatus according to the embodiment of the present application can be integrated into the electronic device. FIG. 5 is a schematic structural diagram of an electronic device according to an embodiment of the present application. As shown in FIG. 5, an electronic device 500 is provided according to this embodiment, which includes: one or more processors 520; a storage apparatus 510 for storing one or more programs, and the one or more programs, when being executed by the one or more processors 520, cause the one or more processors 520 to implement the temperature detection method according to the embodiment of the present application, the method includes the following.

Real-time temperatures of a person is acquired, a temperature changing trend of the person is determined to be a rising trend or a declining trend according to the real-time temperatures, and a real-time temperature changing speed is acquired according to the real-time temperatures.

A higher limit of the real-time temperature changing speed and a lower limit of the real-time temperature changing speed is determined according to the real-time temperature changing speed.

A temperature difference value corresponding to the higher limit of the real-time temperature changing speed is determined according to the higher limit of the real-time temperature changing speed and a temperature difference value corresponding to the lower limit of the real-time temperature changing speed is determined according to the lower limit of the real-time temperature changing speed.

A heat balance temperature higher limit is determined according to the real-time temperatures, the temperature difference value corresponding to the higher limit of the real-time temperature changing speed, and the temperature changing trend; and a heat balance temperature lower limit is determined according to the real-time temperatures, the lower limit of the real-time temperature changing speed, and the temperature changing trend.

The temperature detection result of the person is determined according to a relationship between the heat balance temperature higher limit and an alarm temperature threshold and a relationship between the heat balance temperature lower limit and the alarm temperature threshold.

The processor 520 further implements the technical solution of the temperature detection method according to any embodiment of the present application.

The electronic device 500 shown in FIG. 5 is only an example, and should not limit the functions and scope of use of the embodiments of the present application.

As shown in FIG. 5, the electronic device 500 includes a processor 520, a storage apparatus 510, an input apparatus 530, and an output apparatus 540; the number of processors 520 in the electronic device may be one or more, and in FIG. 5, one processor 520 is taken as an example; the processor 520, the storage apparatus 510, the input apparatus 530 and the output apparatus 540 in the electronic device may be connected through a bus or in other ways. In FIG. 5, the connection through the bus 550 is taken as an example.

The storage apparatus 510, as a computer-readable storage medium, may be used to store software programs, computer-executable programs and module units, such as program instructions corresponding to the temperature detection method in the embodiment of the present application.

The storage apparatus 510 may mainly include a program storage region and a data storage region, where the program storage region may store an operating system and an application program required by at least one function; the data storage region may store data created according to the use of the terminal, and the like. In addition, the storage apparatus 510 may include a high-speed random access memory, and may further include a non-volatile memory, for example, at least one magnetic disk storage device, a flash memory device, or other non-volatile solid-state storage devices. In some examples, the storage apparatus 510 may include a memory arranged remotely from the processor 520, and these remote memories may be connected through a network. Examples of the aforementioned networks include, but are not limited to, the internet, intranets, local area networks, mobile communication networks, and combinations thereof.

The input apparatus 530 may be used to receive input numbers, character information or voice information, and generate key signal inputs related to user settings and function control of the device. The output apparatus 540 may include devices such as a display screen and a speaker.

The electronic device according to an embodiment of the present application may calculate the heat balance temperature value by performing temperature trend detection, and determine the detection result of the human body temperature according to the relationship between the heat balance temperature value and the alarm temperature threshold, so as to achieve the object of improving the detection accuracy of the human body temperature.

The temperature detection apparatus, medium, and electronic device according to the above embodiments may execute the temperature detection method provided in any embodiment of the present application, and have corresponding functional modules for executing the method and achieve corresponding effects which can be achieved through executing the method. For technical details not exhaustively described in the foregoing embodiments, reference may be made to the temperature detection method provided in any embodiment of the present application. relationship between the heat balance temperature lower limit and the alarm temperature threshold.

The processor 520 further implements the technical solution of the temperature detection method according to any embodiment of the present application.

The electronic device 500 shown in FIG. 5 is only an example, and should not limit the functions and scope of use of the embodiments of the present application.

As shown in FIG. 5, the electronic device 500 includes a processor 520, a storage apparatus 510, an input apparatus 530, and an output apparatus 540; the number of processors 520 in the electronic device may be one or more, and in FIG. 5, one processor 520 is taken as an example; the processor 520, the storage apparatus 510, the input apparatus 530 and the output apparatus 540 in the electronic device may be connected through a bus or in other ways. In FIG. 5, the connection through the bus 550 is taken as an example.

The storage apparatus 510, as a computer-readable storage medium, may be used to store software programs, computer-executable programs and module units, such as program instructions corresponding to the temperature detection method in the embodiment of the present application.

The storage apparatus 510 may mainly include a program storage region and a data storage region, where the program storage region may store an operating system and an application program required by at least one function; the data storage region may store data created according to the use of the terminal, and the like. In addition, the storage apparatus 510 may include a high-speed random access memory, and may further include a non-volatile memory, for example, at least one magnetic disk storage device, a flash memory device, or other non-volatile solid-state storage devices. In some examples, the storage apparatus 510 may include a memory arranged remotely from the processor 520, and these remote memories may be connected through a network. Examples of the aforementioned networks include, but are not limited to, the internet, intranets, local area networks, mobile communication networks, and combinations thereof.

The input apparatus 530 may be used to receive input numbers, character information or voice information, and generate key signal inputs related to user settings and function control of the device. The output apparatus 540 may include devices such as a display screen and a speaker.

The electronic device according to an embodiment of the present application may calculate the heat balance temperature value by performing temperature trend detection, and determine the detection result of the human body temperature according to the relationship between the heat balance temperature value and the alarm temperature threshold, so as to achieve the object of improving the detection accuracy of the human body temperature.

The temperature detection apparatus, medium, and electronic device according to the above embodiments may execute the temperature detection method provided in any embodiment of the present application, and have corresponding functional modules for executing the method and achieve corresponding effects which can be achieved through executing the method. For technical details not exhaustively described in the foregoing embodiments, reference may be made to the temperature detection method provided in any embodiment of the present application.

22

**Claims**

1.  A computer-implemented temperature detection method, comprising:

acquiring (S110) real-time temperatures of a person, determining (S110) a temperature changing trend of the person to be a rising trend or a declining trend according to the real-time temperatures, and acquiring (S110) changing speeds of the real-time temperatures;

determining (S120) a higher limit of the real-time temperature changing speed and a lower limit of the real-time temperature changing speed according to the real-time temperature changing speed;

constructing a table of a mapping relationship between magnitudes of the changing speeds and temperature difference values, wherein each of the temperature difference values is a difference between a heat balance temperature and a respective one of the real-time temperatures;

finding a temperature difference value corresponding to the higher limit of the real-time temperature changing speed and a temperature difference value corresponding to the lower limit of the real-time temperature changing speed from the table of the mapping relationship;

in a case where the temperature changing trend is the rising trend, determining that a heat balance temperature higher limit is a sum of a latest one of the real-time temperatures and the temperature difference value corresponding to the higher limit of the real-time temperature changing speed and a heat balance temperature lower limit is the sum of the latest one of the real-time temperatures and the temperature difference value corresponding to the lower limit of the real-time temperature changing speed; and in a case where the temperature changing trend is the declining trend, determining that the heat balance temperature higher limit is a difference between the latest one of the real-time temperatures and the temperature difference value corresponding to the lower limit of the real-time temperature changing speed and the heat balance temperature lower limit is a difference between the latest one of the real-time temperatures and the temperature difference value corresponding to the higher limit of the real-time temperature changing speed;

determining (S150) a temperature detection result of the person according to a relationship between the heat balance temperature higher limit and an alarm temperature threshold and a relationship between the heat balance temperature lower limit and the alarm temperature threshold, which comprises: in a case where the temperature changing trend is the rising trend and the alarm temperature threshold is less than the heat balance temperature lower limit, determining that the temperature detection result is an abnormal high temperature; and in a case where the temperature changing trend is the rising trend and the alarm temperature threshold is greater than the heat balance temperature lower limit, determining that the temperature detection result is a normal body temperature; and in a case where the temperature changing trend is the declining trend and the alarm temperature threshold is less than the heat balance temperature lower limit, determining that the temperature detection result is the abnormal high temperature; and in a case where the temperature changing trend is the declining trend and the alarm temperature threshold is greater than the heat balance temperature higher limit,

**EP 4 177 583 B1**

determining that the temperature detection result is the normal body temperature; wherein the temperature detection result is used to determine whether the person is at normal body temperature.

2. The method according to claim 1, wherein,

before the determining a temperature detection result of the person according to a relationship between the heat balance temperature higher limit and an alarm temperature threshold and a relationship between the heat balance temperature lower limit and the alarm temperature threshold, the method further comprises: finding a balancing time higher limit corresponding to the higher limit of the real-time temperature changing speed and a balancing time lower limit corresponding to the lower limit of the real-time temperature changing speed from the table of the mapping relationship;

the determining the temperature detection result of the person according to the relationship between the heat balance temperature higher limit and the alarm temperature threshold and the relationship between the heat balance temperature lower limit and the alarm temperature threshold comprises:

in a case where the temperature changing trend is the rising trend and the alarm temperature threshold is greater than the heat balance temperature lower limit and less than the heat balance temperature higher limit, according to a speed at which a temperature difference value between the heat balance temperature higher limit and the latest one of the real-time temperatures reaches the balancing time higher limit, calculating a maximum time required for the latest one of the real-time temperatures to rise to the alarm temperature threshold by using a uniform deceleration formula; in a case where the maximum time for the latest one of the real-time temperatures to rise to the alarm temperature threshold is less than or equal to the acceptable measurement time threshold, continuing temperature measurement; and in a case where the maximum time for the latest one of the real-time temperatures to rise to the alarm temperature threshold is greater than the acceptable measurement time threshold, issuing a warning message of temperature instability; and

in a case where the temperature changing trend is the declining trend and the alarm temperature threshold is greater than the heat balance temperature lower limit and less than the heat balance temperature higher limit, according to a speed at which a temperature difference value between the heat balance temperature lower limit and the latest one of the real-time temperatures reaches the balancing time lower limit, and according to the uniform deceleration formula, calculating a maximum time required for the latest one of the real-time temperatures to decline to the alarm temperature threshold by using the uniform deceleration formula; in a case where the maximum time for the latest one of the real-time temperatures to decline to the alarm temperature threshold is less than or equal to the acceptable measurement time threshold, continuing temperature measurement; and in a case where the maximum time for the latest one of the real-time temperatures to decline to the alarm temperature threshold is greater than the acceptable measurement time threshold, issuing a warning message of temperature instability.

3. The method according to claim 1, wherein

the real-time temperature changing speed comprise a temperature increasing speed and a temperature decreasing speed; and

the determining the higher limit of the real-time temperature changing speed and the lower limit of the real-time temperature changing speed according to the real-time temperature changing speed comprises: determining the higher limit of the real-time temperature changing speed according to the products of a preset higher limit coefficient and the temperature increasing speed, and determining the lower limit of the real-time temperature changing speed according to the products of a preset lower limit coefficient and the temperature decreasing speed.

4. The method according to claim 1, wherein the acquiring real-time temperatures of the person and determining the temperature changing trend of the person to be the rising trend or the declining trend according to the real-time temperatures, comprises:

sampling the real-time temperatures of the person according to a preset period;

determining an average slope of real-time temperature sampled points of the person through a preset temperature trend detection window; and

in a case where the average slope is greater than 0, determining that the temperature changing trend is the rising trend; and in a case where the average slope is less than 0, determining that the temperature changing trend is the declining trend.

12

**5.** The method according to claim 4, further comprising:

in a case where the temperature changing trend is the rising trend, calculating a temperature increasing speed in the preset temperature trend detection window and using the temperature increasing speed as the real-time temperature changing speed; and
in a case where the temperature changing trend is the declining trend, calculating a temperature decreasing speed in the preset temperature trend detection window and using the temperature decreasing speed as the real-time temperature changing speed.

**6.** A computer-readable storage medium storing a computer program, wherein a processor, when executing the computer program, implements the temperature detection method according to any one of claims 1 to 5.

**7.** An electronic device, comprising a memory, a processor, and a computer program stored on the memory and operable on the processor, wherein, the processor, when executing the computer program, implements the temperature detection method according to any one of claims 1 to 5.

**Patentansprüche**

**1.** Computerimplementiertes Verfahren zur Temperaturerfassung, umfassend:

Erfassen (S110) von Echtzeit-Temperaturen einer Person, Bestimmen (S110) eines Temperaturänderungstrends der Person als steigenden Trend oder fallenden Trend gemäß den Echtzeit-Temperaturen und Erfassen (S110) von Änderungsgeschwindigkeiten der Echtzeit-Temperaturen;
Bestimmen (S120) einer Obergrenze der Echtzeit-Temperaturänderungsgeschwindigkeit und einer Untergrenze der Echtzeit-Temperaturänderungsgeschwindigkeit in Abhängigkeit von der Echtzeit-Temperaturänderungsgeschwindigkeit;
Erstellen einer Tabelle einer Zuordnungsbeziehung zwischen den Größen der Änderungsgeschwindigkeiten und den Temperaturdifferenzwerten, wobei jeder der Temperaturdifferenzwerte eine Differenz zwischen einer Wärmebilanz-Temperatur und einer jeweiligen der Echtzeit-Temperaturen ist;
Ermitteln eines Temperaturdifferenzwerts, der der Obergrenze der Echtzeit-Temperaturänderungsgeschwindigkeit entspricht, und eines Temperaturdifferenzwerts, der der Untergrenze der Echtzeit-Temperaturänderungsgeschwindigkeit entspricht, aus der Tabelle der Zuordnungsbeziehung;
in einem Fall, in dem der Temperaturänderungstrend der steigende Trend ist, Feststellen, dass eine Wärmebilanz-Temperatur-Obergrenze eine Summe aus einer letzten der Echtzeit-Temperaturen und dem Temperaturdifferenzwert ist, der der Obergrenze der Echtzeit-Temperaturänderungsgeschwindigkeit entspricht, und dass eine Wärmebilanz-Temperatur-Untergrenze die Summe aus der letzten der Echtzeit-Temperaturen und dem Temperaturdifferenzwert ist, der der Untergrenze der Echtzeit-Temperaturänderungsgeschwindigkeit entspricht; und in einem Fall, in dem der Temperaturänderungstrend der fallende Trend ist, Feststellen, dass die Wärmebilanz-Temperatur-Obergrenze eine Differenz zwischen der letzten der Echtzeit-Temperaturen und dem Temperaturdifferenzwert ist, der der Untergrenze der Echtzeit-Temperaturänderungsgeschwindigkeit entspricht, und dass die Wärmebilanz-Temperatur-Untergrenze eine Differenz zwischen der letzten der Echtzeit-Temperaturen und dem Temperaturdifferenzwert ist, der der Obergrenze der Echtzeit-Temperaturänderungsgeschwindigkeit entspricht;
Bestimmen (S150) eines Temperaturerfassungsergebnisses der Person gemäß einer Beziehung zwischen der Wärmebilanz-Temperatur-Obergrenze und einem Alarmtemperaturschwellenwert und einer Beziehung zwischen der Wärmebilanz-Temperatur-Untergrenze und dem Alarmtemperaturschwellenwert, was Folgendes umfasst: in einem Fall, in dem der Temperaturänderungstrend der steigende Trend ist und der Alarmtemperaturschwellenwert unterhalb der Wärmebilanz-Temperatur-Untergrenze liegt, Feststellen, dass das Temperaturerfassungsergebnis eine abnorm hohe Temperatur ist; und in einem Fall, in dem der Temperaturänderungstrend der steigende Trend ist und der Alarmtemperaturschwellenwert oberhalb der Wärmebilanz-Temperatur-Untergrenze liegt, Feststellen, dass das Temperaturerfassungsergebnis eine normale Körpertemperatur ist; und in einem Fall, in dem der Temperaturänderungstrend der fallende Trend ist und der Alarmtemperaturschwellenwert unterhalb der Wärmebilanz-Temperatur-Untergrenze liegt, Feststellen, dass das Temperaturerfassungsergebnis die abnormale hohe Temperatur ist; und in einem Fall, in dem der Temperaturänderungstrend der fallende Trend ist und der Alarmtemperaturschwellenwert oberhalb der Wärmebilanz-Temperatur-Obergrenze liegt, Feststellen, dass das Temperaturerfassungsergebnis die normale Körpertemperatur ist; wobei das Temperaturerfassungsergebnis verwendet wird, um festzustellen, ob die Person eine normale Körpertemperatur hat.

2. Verfahren nach Anspruch 1, wobei

das Verfahren vor dem Bestimmen eines Temperaturerfassungsergebnisses der Person gemäß einer Beziehung zwischen der Wärmebilanz-Temperatur-Obergrenze und einem Alarmtemperaturschwellenwert und einer Beziehung zwischen der Wärmebilanz-Temperatur-Untergrenze und dem Alarmtemperaturschwellenwert ferner Folgendes umfasst: Ermitteln einer Ausgleichszeit-Obergrenze, die der Obergrenze der Echtzeit-Temperaturänderungsgeschwindigkeit entspricht, und einer Ausgleichszeit-Untergrenze, die der Untergrenze der Echtzeit-Temperaturänderungsgeschwindigkeit entspricht, aus der Tabelle der Zuordnungsbeziehung;
das Bestimmen des Temperaturerfassungsergebnisses der Person gemäß der Beziehung zwischen der Wärmebilanz-Temperatur-Obergrenze und dem Alarmtemperaturschwellenwert und der Beziehung zwischen der Wärmebilanz-Temperatur-Untergrenze und dem Alarmtemperaturschwellenwert Folgendes umfasst:

in einem Fall, in dem der Temperaturänderungstrend der steigende Trend ist und der Alarmtemperaturschwellenwert oberhalb der Wärmebilanz-Temperatur-Untergrenze und unterhalb der Wärmebilanz-Temperatur-Obergrenze liegt, Berechnen einer maximalen Zeit, die erforderlich ist, damit die letzte der Echtzeit-Temperaturen auf den Alarmtemperaturschwellenwert ansteigt, unter Verwendung einer gleichmäßigen Verzögerungsformel, gemäß einer Geschwindigkeit, bei der ein Temperaturdifferenzwert zwischen der Wärmebilanz-Temperatur-Obergrenze und der letzten der Echtzeit-Temperaturen die Ausgleichszeit-Obergrenze erreicht; in einem Fall, in dem die maximale Zeit, die die letzte der Echtzeit-Temperaturen benötigt, um auf den Alarmtemperaturschwellenwert anzusteigen, kleiner oder gleich dem akzeptablen Messzeit-Schwellenwert ist, Fortsetzen der Temperaturmessung; und in einem Fall, in dem die maximale Zeit, die die letzte der Echtzeit-Temperaturen benötigt, um auf den Alarmtemperaturschwellenwert anzusteigen, größer als der akzeptable Messzeit-Schwellenwert ist, Ausgeben einer Warnmeldung über eine Temperaturinstabilität; und

in einem Fall, in dem der Temperaturänderungstrend der fallende Trend ist und der Alarmtemperaturschwellenwert oberhalb der Wärmebilanz-Temperatur-Untergrenze und unterhalb der Wärmebilanz-Temperatur-Obergrenze liegt, Berechnen einer maximalen Zeit, die erforderlich ist, damit die letzte der Echtzeit-Temperaturen auf den Alarmtemperaturschwellenwert absinkt, unter Verwendung der gleichmäßigen Verzögerungsformel, gemäß einer Geschwindigkeit, bei der ein Temperaturdifferenzwert zwischen der Wärmebilanz-Temperatur-Untergrenze und der letzten der Echtzeit-Temperaturen die Ausgleichszeit-Untergrenze erreicht, und gemäß der gleichmäßigen Verzögerungsformel; in einem Fall, in dem die maximale Zeit, die die letzte der Echtzeit-Temperaturen benötigt, um auf den Alarmtemperaturschwellenwert abzusinken, kleiner oder gleich dem akzeptablen Messzeit-Schwellenwert ist, Fortsetzen der Temperaturmessung; und in einem Fall, in dem die maximale Zeit, die die letzte der Echtzeit-Temperaturen benötigt, um auf den Alarmtemperaturschwellenwert abzusinken, größer als der akzeptable Messzeit-Schwellenwert ist, Ausgeben einer Warnmeldung über eine Temperaturinstabilität.

3. Verfahren nach Anspruch 1, wobei

die Echtzeit-Temperaturänderungsgeschwindigkeit eine Temperaturanstiegsgeschwindigkeit und eine Temperaturabfallgeschwindigkeit umfasst; und
das Bestimmen der Obergrenze der Echtzeit-Temperaturänderungsgeschwindigkeit und der Untergrenze der Echtzeit-Temperaturänderungsgeschwindigkeit in Abhängigkeit von der Echtzeit-Temperaturänderungsgeschwindigkeit Folgendes umfasst: Bestimmen der Obergrenze der Echtzeit-Temperaturänderungsgeschwindigkeit gemäß den Produkten eines voreingestellten Obergrenzenkoeffizienten und der Temperaturanstiegsgeschwindigkeit, und Bestimmen der Untergrenze der Echtzeit-Temperaturänderungsgeschwindigkeit gemäß den Produkten eines voreingestellten Untergrenzenkoeffizienten und der Temperaturabfallgeschwindigkeit.

4. Verfahren nach Anspruch 1, wobei das Erfassen der Echtzeit-Temperaturen der Person und das Bestimmen des Temperaturänderungstrends der Person als steigender Trend oder fallender Trend gemäß den Echtzeit-Temperaturen Folgendes umfassen:

Abtasten der Echtzeit-Temperaturen der Person gemäß einem voreingestellten Zeitraum;
Bestimmen einer durchschnittlichen Steigung der Echtzeit-Temperatur-Abtastpunkte der Person durch ein voreingestelltes Temperaturtrenderfassungsfenster; und
in einem Fall, in dem die durchschnittliche Steigung größer als 0 ist, Feststellen, dass der Temperaturänderungstrend der steigende Trend ist; und in einem Fall, in dem die durchschnittliche Steigung kleiner als 0 ist, Feststellen, dass der Temperaturänderungstrend der fallende Trend ist.

**5.** Verfahren nach Anspruch 4, umfassend ferner:

in einem Fall, in dem der Temperaturänderungstrend der steigende Trend ist, Berechnen einer Temperaturanstiegsgeschwindigkeit in dem voreingestellten Temperaturtrenderfassungsfenster und Verwenden der Temperaturanstiegsgeschwindigkeit als Echtzeit-Temperaturänderungsgeschwindigkeit; und
in einem Fall, in dem der Temperaturänderungstrend der fallende Trend ist, Berechnen einer Temperaturabfallgeschwindigkeit in dem voreingestellten Temperaturtrenderfassungsfenster und Verwenden der Temperaturabfallgeschwindigkeit als Echtzeit-Temperaturänderungsgeschwindigkeit.

**6.** Computerlesbares Speichermedium, auf dem ein Computerprogramm gespeichert ist, wobei ein Prozessor bei der Ausführung des Computerprogramms das Temperaturerfassungsverfahren nach einem der Ansprüche 1 bis 5 implementiert.

**7.** Elektronisches Gerät, umfassend einen Speicher, einen Prozessor und ein auf dem Speicher gespeichertes und auf dem Prozessor ausführbares Computerprogramm, wobei der Prozessor bei der Ausführung des Computerprogramms das Temperaturerfassungsverfahren nach einem der Ansprüche 1 bis 5 implementiert.

**Revendications**

**1.** Procédé de détection de température mis en œuvre par ordinateur, comprenant :

acquérir (S110) en temps réel les températures d'une personne, déterminer (S110) une tendance de changement de température de la personne comme tendance montante ou tendance descendante en fonction des températures en temps réel, et acquérir (S110) les vitesses de changement des températures en temps réel ;
déterminer (S120) une limite supérieure de la vitesse de changement de température en temps réel et une limite inférieure de la vitesse de changement de température en temps réel en fonction de la vitesse de changement de température en temps réel ;
construire une tableau de relation de mappage entre les magnitudes des vitesses de changement et les valeurs de différence de température, dans lequel chacune des valeurs de différence de température est une différence entre une température d'équilibre thermique et l'une respective des températures en temps réel ;
trouver la valeur de différence de température correspondant à la limite supérieure de la vitesse de changement de température en temps réel et la valeur de différence de température correspondant à la limite inférieure de la vitesse de changement de température en temps réel à partir du tableau de relation de mappage ;
dans le cas où la tendance de changement de température est la tendance montante, déterminer que la limite supérieure de température d'équilibre thermique est la somme de la dernière des températures en temps réel et de la valeur de différence de température correspondant à la limite supérieure de la vitesse de changement de température en temps réel et la limite inférieure de température d'équilibre thermique est la somme de la dernière des températures en temps réel et de la valeur de différence de température correspondant à la limite inférieure de la vitesse de changement de température en temps réel ; et dans le cas où la tendance de changement de température est la tendance descendante, déterminer que la limite supérieure de température d'équilibre thermique est la différence entre la dernière des températures en temps réel et la valeur de différence de température correspondant à la limite inférieure de la vitesse de changement de température en temps réel et la limite inférieure d'équilibre thermique est la différence entre la dernière des températures en temps réel et la valeur de différence de température correspondant à la limite supérieure de la vitesse de changement de température en temps réel ;
déterminer (S150) le résultat de détection de température de la personne en fonction de la relation entre la limite supérieure de température d'équilibre thermique et un seuil de température d'alerte et la relation entre la limite inférieure de température d'équilibre thermique et le seuil de température d'alerte, qui comprend : dans le cas où la tendance de changement de température est la tendance montante et le seuil de température d'alerte est inférieur à la limite inférieure de température d'équilibre thermique, déterminer que le résultat de détection de température est une température anormalement élevée ; et dans le cas où la tendance de changement de température est la tendance montante et le seuil de température d'alerte est supérieur à la limite inférieure de température d'équilibre thermique, déterminer que le résultat de détection de température est une température corporelle normale ; et dans le cas où la tendance de changement de température est la tendance descendante et le seuil de température d'alerte est inférieur à la limite inférieure de température d'équilibre thermique, déterminer que le résultat de détection de température est une température anormalement élevée ; et dans le cas où la tendance de changement de température est la tendance descendante et le seuil de température d'alerte est supérieur à la limite supérieure de température d'équilibre thermique, déterminer que le résultat de détection de température est une température corporelle normale ; dans lequel le résultat de détection de température est utilisé pour déterminer si la personne est à une température corporelle normale.

2. Procédé selon la revendication 1, dans lequel,

avant de déterminer un résultat de détection de température de la personne en fonction de la relation entre la limite supérieure de température d'équilibre thermique et un seuil de température d'alerte et de la relation entre la limite inférieure de température d'équilibre thermique et le seuil de température d'alerte, le procédé comprend en outre : trouver une limite supérieure de temps d'équilibre correspondant à la limite supérieure de la vitesse de changement de température en temps réel et une limite inférieure de temps d'équilibre correspondant à la limite inférieure de la vitesse de changement de température en temps réel à partir du tableau de la relation de mappage ;
déterminer le résultat de détection de température de la personne en fonction de la relation entre la limite supérieure de température d'équilibre thermique et le seuil de température d'alerte et la relation entre la limite inférieure de température d'équilibre thermique et le seuil de température d'alerte comprend :

dans le cas où la tendance de changement de température est la tendance montante et le seuil de température d'alerte est supérieur à la limite inférieure de température d'équilibre thermique et inférieur à la limite supérieure de température d'équilibre thermique, en fonction d'une vitesse à laquelle une valeur de différence de température entre la limite supérieure de température d'équilibre thermique et la dernière des températures en temps réel atteint la limite supérieure de temps d'équilibre, calculer la durée maximale requise pour que la dernière des températures en temps réel monte au seuil de température d'alerte en utilisant une formule de décélération uniforme ; dans le cas où la durée maximale pour que la dernière des températures en temps réel monte au seuil de température d'alerte est inférieure ou égale au seuil de durée de mesure acceptable, poursuivre la mesure de température ; et dans le cas où la durée maximale pour que la dernière des températures en temps réel monte au seuil de température d'alerte est supérieure au seuil de durée de mesure acceptable, émettre un message d'avertissement d'instabilité de température ; et
dans le cas où la tendance de changement de température est la tendance descendante et le seuil de température d'alerte est supérieur à la limite inférieure de température d'équilibre thermique et inférieur à la limite supérieure de température d'équilibre thermique, en fonction de la vitesse à laquelle la valeur de différence de température entre la limite inférieure de température d'équilibre thermique et la dernière des températures en temps réel atteint la limite inférieure de temps d'équilibre, et en fonction de la formule de décélération uniforme, calculer la durée maximale requise pour que la dernière des températures en temps réel descende au seuil de température d'alerte en utilisant la formule de décélération uniforme ; dans le cas où la durée maximale pour que la dernière des températures en temps réel descende au seuil de température d'alerte est inférieure ou égale au seuil de durée de mesure acceptable, poursuivre la mesure de température ; et dans le cas où la durée maximale pour que la dernière des températures en temps réel descende au seuil de température d'alerte est supérieure au seuil de durée de mesure acceptable, émettre un message d'avertissement d'instabilité de température.

3. Procédé selon la revendication 1, dans lequel

la vitesse de changement de température en temps réel comprend une vitesse d'augmentation de la température et une vitesse de diminution de la température ; et
déterminer la limite supérieure de la vitesse de changement de température en temps réel et la limite inférieure de la vitesse de changement de température en temps réel en fonction de la vitesse de changement de température en temps réel comprend : déterminer la limite supérieure de la vitesse de changement de température en temps réel en fonction des produits d'un coefficient de limite supérieure prédéfini et de la vitesse d'augmentation de la température, et déterminer la limite inférieure de la vitesse de changement de température en temps réel en fonction des produits d'un coefficient de limite inférieure prédéfini et de la vitesse de diminution de la température.

4. Procédé selon la revendication 1, dans lequel acquérir en temps réel les températures d'une personne et déterminer une tendance de changement de température de la personne comme tendance montante ou tendance descendante en fonction des températures en temps réel, comprend :
échantillonner les températures en temps réel de la personne selon une période prédéfinie :

déterminer une pente moyenne de points échantillonnés de température en temps réel de la personne via une fenêtre de détection de tendance de température prédéfinie ; et

dans le cas où la pente moyenne est supérieure à 0, déterminer que la tendance de changement de température est la tendance montante ; et dans le cas où la pente moyenne est inférieure à 0, déterminer que la tendance de changement de température est la tendance descendante.

**5.** Procédé selon la revendication 4, comprenant en outre :

dans le cas où la tendance de changement de température est la tendance montante, calculer une vitesse d'augmentation de la température dans la fenêtre de détection de tendance de température prédéfinie et utiliser la vitesse d'augmentation de la température comme vitesse de changement de température en temps réel ; et dans le cas où la tendance de changement de température est la tendance descendante, calculer une vitesse de diminution de la température dans la fenêtre de détection de tendance de température prédéfinie et utiliser la vitesse de diminution de la température comme vitesse de changement de température en temps réel.

**6.** Support de stockage lisible par ordinateur stockant un programme informatique, dans lequel un processeur, lorsqu'il exécute le programme informatique, met en œuvre le procédé de détection selon l'une quelconque des revendications 1 à 5.

**7.** Dispositif électronique, comprenant une mémoire, un processeur, et un programme informatique stocké dans la mémoire et pouvant être exécuté sur le processeur, dans lequel, le processeur, lorsqu'il exécute le programme informatique, met en œuvre le procédé de détection selon l'une quelconque des revendications 1 à 5.

Acquire real-time temperatures of a detected person, determine a temperature changing trend of the detected person to be a rising trend or a declining trend, and acquire a real-time temperature changing speed    S110

Determine a higher limit of the real-time temperature changing speed and a lower limit of the real-time temperature changing speed according to the real-time temperature changing speed    S120

Determine a temperature difference value corresponding to the higher limit of the real-time temperature changing speed according to the higher limit of the real-time temperature changing speed, and determine a temperature difference value corresponding to the lower limit of the real-time temperature changing speed according to the lower limit of the real-time temperature changing speed    S130

Determine a heat balance temperature higher limit according to the real-time temperatures, the temperature difference value corresponding to the higher limit of the real-time temperature changing speed, and the temperature changing trend; determine a heat balance temperature lower limit according to the real-time temperatures, the lower limit of the real-time temperature changing speed, and the temperature changing trend    S140

Determine a temperature detection result of the detected person according to a relationship between the heat balance temperature higher limit and an alarm temperature threshold and a relationship between the heat balance temperature lower limit and the alarm temperature threshold    S150

**FIG. 1**

**FIG. 2**

**FIG. 3**

410

Real-time
temperature
changing speed
acquisition module

420

Threshold
determination
module

430

Temperature
difference value
determination
module

450

Detection
result
determination
module

440

Heat balance
temperature range
determination
module

**FIG. 4**

500

Storage
apparatus

510

Input apparatus

530

Output apparatus

540

550

Processor

520

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4843577 A, MURAMOTO YUTAKA [JP]
- US 19890627 A
- CN 1584523 A, OMRON HEALTHCARE CO LTD [JP]
- CN 20050223 A
- CN 105371968 A
- CN 20160302 A